# EUROPÄISCHE PATENTSCHRIFT

(11) **EP 3 558 146 B1**
(45) Veröffentlichungstag und Bekanntmachung des Hinweises auf die Patenterteilung: **31.03.2021**
(21) Anmeldenummer: 19704226.0
(22) Anmeldetag: 25.01.2019
(51) Int. Cl.: A61B 17/80, A61F 2/34, A61F 2/30

(54) **MEDIZINISCHE KNOCHENSCHRAUBE UND IMPLANTATSYSTEM**
MEDICAL BONE SCREW AND IMPLANT SYSTEM
VIS D'OSTÉOSYNTHÈSE MÉDICALE ET SYSTÈME D'IMPLANT

(30) Priorität: 25.01.2018 DE 102018101657
(43) Veröffentlichungstag der Anmeldung: 30.10.2019
(73) Patentinhaber: Aesculap AG, 78532 Tuttlingen (DE)
(72) Erfinder: LINDNER, Stephan, 78573 Wurmlingen (DE); SCHNEIDER, Jens, 78315 Radolfzell (DE)
(74) Vertreter: Winter, Brandl - Partnerschaft mbB
(86) Internationale Anmeldenummer: PCT/EP2019/051880
(87) Internationale Veröffentlichungsnummer: WO 2019/145490

(56) Entgegenhaltungen:
- WO-A1-2004/082493
- US-A- 4 955 919
- US-A- 5 725 588
- US-A1- 2004 068 319
- US-A1- 2014 324 108

## Beschreibung

### Gebiet der Erfindung

Die Erfindung betrifft ein Implantatsystem mit mindestens einer medizinischen Knochenschraube und einem Hüftgelenkspfannenimplantat, das mindestens ein Durchgangsloch aufweist, durch welches die medizinische Knochenschraube derart einführbar ist, dass die medizinische Knochenschraube zumindest in eine Richtung des Durchgangslochs schwenkbar ist.

### Hintergrund der Erfindung

Zur Verankerung von Hüftgelenkspfannenimplantaten / künstlichen Hüftgelenkspfannen im Beckenknochen werden die einzusetzenden Hüftgelenkspfannenimplantate häufig mittels Knochenschrauben fixiert, insbesondere bei Revisionseingriffen oder primärer starker Destruktion des Hüftgelenkes auf der Beckenseite. Um eine stabile Verankerung der Knochenschrauben im Beckenknochen zu ermöglichen, müssen die Knochenschrauben an einer Stelle im Beckenknochen platziert werden, wo genug und ausreichend tragfähiger Knochen vorgefunden wird. Um beim Verschrauben des Hüftgelenkspfannenimplantats mit dem Hüftknochen die Platzierung der Knochenschraube an einer solchen Stelle zu ermöglichen, ist ein möglichst hoher Freiheitsgrad der Knochenschraube sowohl hinsichtlich des Knochenschraubeneintrittspunkts in den Knochen als auch hinsichtlich der Angulation / Winkelung der Schraubenachse in Bezug auf die Außengeometrie bzw. den Normalvektor der Außengeometrie des Hüftgelenkspfannenimplantats erforderlich.

Bei dünnwandigen Hüftgelenkspfannenimplantaten kann jedoch bereits eine geringe Neigung der Knochenschraube im vorgesehenen Loch des Hüftgelenkspfannenimplantats zur Folge haben, dass der Knochenschraubenkopf aufgrund der Auswinkelung in das Innere des Hüftgelenkspfannenimplantats hineinragt und ein dort zur Anlage bringendes Inlay auf dem Knochenschraubenkopf aufsitzt, wodurch eine stabile Verankerung des Inlays in dem Hüftgelenkspfannenimplantat verhindert wird und es zu einem frühen Implantatversagen kommen kann.

Weiter besteht an der Verbindungsstelle von Knochenschraube und Hüftgelenkspfannenimplantat die Gefahr eines Durchschraubens beim Anziehen der Knochenschraube, sodass diese Verbindungsstelle robust ausgelegt sein muss. Darüber hinaus kann es beim Anziehen der Knochenschraube dazu kommen, dass der Schraubendreher sich durch Verformung des Knochenschraubenkopfes verklemmt und die Knochenschraube möglicherweise ungewollt aus dem Knochenverbund gerissen wird oder ein Wiedereinsetzen des Schraubendrehers erschwert ist.

### Stand der Technik

Bekannt ist aus dem Stand der Technik ein Hüftgelenkspfannenimplantat, das der Knochenschraube durch spezielle Schraubenlager, die an der Außenseite des Hüftgelenkspfannenimplantats hervorstehen und konische Bohrungen runden Querschnitts aufweisen, mehr Winkelspielraum ermöglicht. Die Fertigung eines solchen Hüftgelenkspfannenimplantats ist aufgrund der hervorstehenden Schraubenlager jedoch zum einen aufwendig und bringt einen erhöhten Aufwand in der OP-Technik mit sich, zum anderen kann der Knochenschraubeneintrittspunkt nur innerhalb des Schwenkkreises um den Mittelpunkt der runden Bohrung platziert werden.

Aus der Patentschrift US 9 655 665 B2 ist ein Implantatsystem bekannt, das für eine Knochenschraube eine Unterlegscheibe runden Querschnitts vorsieht, die an der Außenseite ihres umlaufenden Rands zwei Zapfen aufweist, an denen die Unterlegscheibe drehbar in einer komplementär ausgebildeten Ausnehmung des Implantats lagerbar ist. Dabei sind die Lagerpositionen der Zapfen im Implantat festgelegt, sodass eine auf diese Unterlegscheibe aufgeschraubte Knochenschraube nur im Umkreis um die Drehachse der Unterlegscheibe positionierbar ist.

Die Patentschrift US 9 662 145 B2 zeigt ein Implantatsystem mit einer Knochenschraube und einer unrunden Unterlegscheibe für ein Langloch, mit der die Knochenschraube gegen Durchschrauben gesichert werden kann, die jedoch ein nachteiliges Hervorstehen des Schraubenkopfes in das Innere des Implantats bei Neigung der Knochenschraube nicht verhindern kann.

In US 5,725,588 ist ein Implantatsystem offenbart mit einer Hüftgelenkspfanne, einem Kupplungselement und einer Knochenschraube, die einen Kopfteil und einen Schaftabschnitt mit Gewinde aufweist. Das Kupplungselement weist zumindest einen axialen Schlitz auf, der eine Aufdehnung des Kupplungselements in radialer Richtung ermöglicht, damit der Kopfteil der Knochenschraube mit dem Kopfteil voran in das Kupplungselement eingesteckt werden kann. Die Endsicherung der Kopfteil-Kupplungselement-Konstruktion erfolgt erst durch Einsetzen dieser in ein Einsatzloch in der Hüftgelenkspfanne, wodurch eine erneute Aufdehnung des Kupplungselements verhindert wird.

US 2004/0068319 A1 offenbart eine zervikale Knochenplatte mit mehreren Durchgangslöchern, in die jeweils eine Schraube-Unterlegscheibe-Konstruktion einsetzbar ist. Dabei kann die Unterlegscheibe im Durchgangsloch um eine Achse verschwenkt werden, die durch Vorsprünge in radialer Richtung an der Unterlegscheibe selbst definiert ist.

### Kurze Beschreibung der Erfindung

Es ist daher Aufgabe der Erfindung, die Nachteile aus dem Stand der Technik zu beseitigen oder zumindest zu mildern. Insbesondere soll beim Verschrauben eines Hüftgelenkspfannenimplantats an einem Patientenknochen ermöglicht werden, die Gesamtposition bzw. die Anordnung der Knochenschraube im Knochen möglichst frei wählen zu können, also sowohl den Knochenschraubeneintrittspunkt als auch die Winkelung der Knochenschraube hinsichtlich der Außengeometrie des Hüftgelenkspfannenimplantats, um dadurch der Knochenschraube im Knochen idealen Halt zu bieten. Insbesondere soll weiter ein Aufsitzen eines Hüftgelenkspfannenimplantat-Inlays auf dem Kopf einer hinsichtlich der Außengeometrie des Hüftgelenkspfannenimplantats abgewinkelten Knochenschraube verhindert oder zumindest reduziert werden und außerdem ein Durchschrauben der Knochenschraube an der Verbindungsstelle zwischen Hüftgelenkspfannenimplantat und Knochenschraube verhindert werden. Weiter soll insbesondere ein Verklemmen des Schraubendrehers im Kopf der Knochenschraube beim Anziehen der Knochenschraube verhindert werden.

Diese Aufgabe wird erfindungsgemäß gelöst durch die Merkmalskombination des Anspruchs 1. Vorteilhafte Weiterbildungen der Erfindung sind Gegenstand der beigefügten Unteransprüche.

Ein Grundgedanke der Erfindung besteht darin, zur Verschraubung des Hüftgelenkspfannenimplantats am Patientenknochen eine Knochenschraube mit einem gelenkigen Kopf zu schaffen, der dem Knochenschraubenschaft der Knochenschraube bei der Ausrichtung relativ zum Hüftgelenkspfannenimplantat mindestens einen zusätzlichen Freiheitsgrad gewährt und/oder eine unveränderte Anlageposition des Schraubenkopfes am Hüftgelenkspfannenimplantat ermöglicht, wenn der Knochenschraubenschaft relativ zum Hüftgelenkspfannenimplantat abgewinkelt wird. Im Hüftgelenkspfannenimplantat ist mindestens ein Durchgangsloch, vorzugsweise Langloch, vorgesehen, bevorzugt zwei oder mehr, in das die Knochenschraube derart einführbar ist, dass die Knochenschraube in zumindest eine Richtung des Durchgangslochs schwenkbar ist, vorzugsweise entlang der Längsachse des Langlochs frei platzierbar und in Richtung der Längsachse schwenkbar ist.

Im Konkreten wird ein Implantatsystem vorgeschlagen mit mindestens einer medizinischen Knochenschraube und einem Hüftgelenkspfannenimplantat, das mindestens ein Durchgangsloch, vorzugsweise ein Langloch mit zwei sich gegenüberliegenden langen Längsseiten und zwei sich gegenüberliegenden kurzen Querseiten, aufweist und durch welches die medizinische Knochenschraube derart einführbar ist, dass die medizinische Knochenschraube zumindest in eine Richtung des Durchgangslochs schwenkbar ist, vorzugsweise zumindest in Längsrichtung des Langlochs schwenkbar ist, besonders bevorzugt in Längsrichtung und Querrichtung des Durchgangslochs schwenkbar ist. Dabei weist die medizinische Knochenschraube zur Befestigung des Hüftgelenkspfannenimplantats an einem Patientenknochen einen schaftförmigen Knochengewindeteil auf, der einen Kerndurchmesser (bspw. 3mm) und einen Außengewindedurchmesser (bspw. 6,5mm) hat und an dessen proximalem Ende sich ein zweigeteilter, winkelbarer Kopfteil anschließt. Weiter ist der zweigeteilte, winkelbare Kopfteil aus einem (stoff)einstückig mit dem Knochengewindeteil ausgebildeten, vorzugsweise rotationssymmetrischen, weiter vorzugsweise teilkugelförmigen oder vollständig kugelförmigen Kopfstück und einem separat zu dem Knochengewindeteil vorgesehenen, vorzugsweise nicht-rotationssymmetrischen Kopfstück gebildet. Dabei weist das separat zu dem Knochengewindeteil vorgesehene Kopfstück eine Oberseite und eine Unterseite auf und ein Durchgangsloch ist mit einem Hinterschnitt bzw. einer Trichter- oder Tulpenform in dieses eingebracht. Das Durchgangsloch hat weiter einen proximalen Durchgangslochdurchmesser an der Oberseite, einen distalen Durchgangslochdurchmesser (bspw. 4,2mm) an der Unterseite und zwischen der Oberseite und der Unterseite ein dem Außengewinde des schaftförmigen Knochengewindeteils entsprechendes Innengewinde mit einem Innengewindedurchmesser, wobei der Innengewindedurchmesser größer als der Außengewindedurchmesser und der distale Durchgangslochdurchmesser größer als der Kerndurchmesser ist. Weiter ist der schaftförmige Knochengewindeteil durch Einschrauben des schaftförmigen Knochengewindeteils in das Durchgangsloch derart in das separat zu dem Knochengewindeteil vorgesehene Kopfstück eingeführt, , dass das (stoff)einstückig mit diesem ausgebildete Kopfstück an dem Hinterschnitt bzw. in der Trichter- oder Tulpenform axialfest anliegt, jedoch eine Schwenkbewegung des schaftförmigen Knochengewindeteils bezüglich des separaten Kopfstücks in zumindest einer oder ausschließlich einer Schwenkebene zugelassen ist.

Dabei kann die Öffnung bzw. die Innenumfangsseite / Umfangsfläche der Trichterform des Durchgangslochs in Axialrichtung des Durchgangslochs (in Durchtrittsrichtung des Gewindeteils durch das Durchgangsloch) einen linearen und/oder konvexen Verlauf einnehmen bzw. kann sich der Durchmesser (der Trichterform) des Durchgangslochs in Axialrichtung des Durchgangslochs linear und/oder exponentiell (konvex) verjüngen.

In anderen Worten ist zum Verschrauben eines Hüftgelenkspfannenimplantats an einem Patientenknochen eine Knochenschraube mit einem Kopfteil vorgesehen, das aus zwei Untereinheiten besteht, die in montiertem Zustand eine gelenkige Verbindung, insbesondere nach Art eines Kugelgelenks, bilden und dem distal zum Kopfteil angeordneten Gewindeschaftteil eine rotatorische Bewegung relativ zum Kopfteil in mindestens einer Ebene, insbesondere nach Art eines Kugelgelenks, gewähren. Dabei ist die erste Untereinheit des Kopfteils fest mit dem Gewindeschaftteil verbunden, bevorzugt einstückig verbunden und besonders bevorzugt stoffeinstückig/integral verbunden, und insbesondere nach Art eines Gelenkkopfes ausgebildet. Die zweite Untereinheit des Kopfteils weist einen Durchbruch mit einer Innengeometrie auf, die (teil)komplementär zur Außengeometrie der ersten Untereinheit ausgebildet ist, derart, dass die zweite Untereinheit die erste Untereinheit im montierten Zustand zumindest teilweise umschließt und mit dieser eine gelenkige Verbindung eingeht. Zur Montage der beiden Untereinheiten wird der Gewindeschaftteil der Knochenschraube durch den Durchbruch der zweiten Untereinheit geführt, sodass die erste Untereinheit an ihrem Außenumfang mit der zweiten Untereinheit an ihrem Innenumfang in Anlage kommt. Insbesondere ist die zweite Untereinheit nach Art einer Gelenkpfanne/Kugelpfanne gebildet.

Alternativ zum Innengewinde sind ähnliche Ausgestaltungen der Innenumfangsfläche des Durchgangslochs denkbar, in denen die Umfangsfläche des Durchgangslochs (eine) profilierte Ausnehmung(en) aufweist, die komplementär zum Außenprofil des schaftförmigen Teils der Knochenschraube beschaffen ist/sind, und mittels derer der schaftförmigen Teil der Knochenschraube einführbar ist. Beispielsweise kann das Durchgangsloch umfangsseitig ein Labyrinth aufweisen.

Durch das Gewinde bzw. die profilierte(n) Ausnehmung(en) ist/sind nur beabsichtigte Bewegungen des separaten Kopfstücks entlang des Knochengewindeteils bzw. schaftförmigen Teils der Knochenschraube in Einschraubrichtung bzw. Einführrichtung möglich, wohingegen es zu einem unbeabsichtigten Abrutschen / Verrutschen / Bewegen des separaten Kopfstücks entlang des Gewindeteils nicht kommen kann.

Es sei angemerkt, dass im Rahmen dieser Patentanmeldung unter dem Ausdruck "proximal" stets ein dem Anwender (Chirurg) zugewandtes bzw. dem Patienten abgewandtes Ende oder Abschnitt zu verstehen ist und entsprechend unter dem Ausdruck "distal" ein dem Anwender abgewandtes bzw. dem Patienten zugewandtes Ende oder Abschnitt zu verstehen ist.

Durch einen solchen zweigeteilten Kopfteil einer Knochenschraube wird vorteilhafterweise erreicht, dass der Kopfteil bezüglich seiner Umgebung, im vorliegenden Fall am/im Durchgangsloch (Langloch) des Hüftgelenkspfannenimplantats (nachfolgend als Implantat bezeichnet), eine feste Position einnehmen / festgelegt werden kann, während der schaftförmige Knochengewindeteil (nachfolgend als Gewindeteil bezeichnet) bezüglich der Umgebung, beispielsweise der Außengeometrie des Implantats, bewegbar ist. Es ist dadurch also möglich, die Anlageposition des Kopfteils am/im Implantat genau zu bestimmen und dem Gewindeteil dennoch eine Vielfalt an Ausrichtungsmöglichkeiten zu gewähren.

Darüber hinaus bietet das Langloch vorteilhafterweise entlang seiner Längsachse eine Vielzahl an Positionsmöglichkeiten für den Kopfteil der Schraube bzw. den Gewindeteil der Knochenschraube, der durch den gelenkigen Kopfteil der Knochenschraube zusätzlich abgewinkelt werden kann. Somit können zum einen der Knochenschraubeneintrittspunkt in den Patientenknochen und zum anderen der Erstreckungswinkel der Knochenschraube relativ zur Außengeometrie des Implantats (Normalvektor der Außengeometrie) in den Patientenknochen frei gewählt werden.

Bevorzugt hat das (stoff)einstückig mit dem Gewindeteil ausgebildete Kopfstück (nachfolgend als verbundenes Kopfstück bezeichnet) einen Kopfstückaußendurchmesser und hat das Durchgangsloch einen intermediären Durchgangslochdurchmesser zwischen der Oberseite und der Unterseite, der größer, gleich oder kleiner als der Kopfstückaußendurchmesser ist. In anderen Worten können also das verbundene Kopfstück und das separate Kopfstück in montiertem Zustand des Kopfteils in einer Spielpassung oder einer Presspassung zueinander stehen. Der intermediäre Durchgangslochdurchmesser bezeichnet dabei den größten Durchmesser in einem Mittenabschnitt des Durchgangslochs, der sich zwischen einem proximalen Abschnitt des Durchgangslochs, der den proximalen Durchgangslochdurchmesser aufweist, und einem distalen Abschnitt des Durchgangslochs, der den distalen Durchgangslochdurchmesser aufweist, befindet.

Es ist auch denkbar, dass das separate Kopfstück und das verbundene Kopfstück einen (leichten) Presssitz bilden. Insbesondere ist der proximale Durchgangslochdurchmesser kleiner als der Kopfstückaußendurchmesser. Die Montage des separaten Kopfstücks auf dem verbundenen Kopfstück kann durch Aufschnappen des separaten Kopfstücks erfolgen. Dadurch wird eine Verliersicherung erreicht bzw. wird das separate Kopfstück verliersicher am verbundenen Kopfstück bzw. am Gewindeteil gehalten.

Weiter ist es möglich, dass im Falle eines vorgesehenen Presssitzes das separate Kopfstück erwärmt wird, so dass sich das Durchgangsloch auf einen Durchmesser aufweitet, durch den der (gekühlte) Gewindeteil einführbar ist. Nach Abkühlen des separaten Kopfstücks, also nach Verengung des Durchmessers des Durchgangslochs, kann der Gewindeteil nicht mehr aus dem separaten Kopfstück herausgezogen werden.

Es ist auch denkbar, dass der Gewindeteil und das damit verbundene Kopfstück erst bei der Montage mit dem separaten Kopfstück aneinander gefügt werden. Konkret bedeutet dies, dass das separate Kopfstück und das mit dem Gewindeteil (einstückig) zu verbindende Kopfstück zunächst vormontiert werden, so dass sie ihre Montageposition relativ zueinander einnehmen. Der Gewindeteil wird anschließend an das zu verbindende Kopfstück angefügt, während das separate Kopfstück bereits in Anlage mit dem zu verbindenden Kopfstück steht und somit zwischen dem Gewindeteil und dem verbundenen Kopfstück beweglich gehalten wird. Ebenso kann eine Vormontage des Gewindeteils mit dem separaten Kopfstück erfolgen und das zu verbindende Kopfstück anschließend an den Gewindeteil gefügt werden. Die Verbindung von Gewindeteil und dem zu verbindenden Kopfstück erfolgt beispielsweise durch Schweißen.

Bevorzugt ist der proximale Durchgangslochdurchmesser größer als der distale Durchgangslochdurchmesser ist und/oder der Hinterschnitt bzw. die Trichter- oder Tulpenform des Durchgangslochs so ausgebildet und/oder ausgerichtet, dass sich zwangsläufig (nur) eine Einführrichtung des Gewindeteils, nämlich von der Oberseite aus, ergibt.

Konkret kann die Einführrichtung dadurch festgelegt werden, dass das Durchgangsloch (nur) an der Oberseite des separaten Kopfstücks einen Durchmesser aufweist, der ausreichend groß ist, um auch das verbundene Kopfstück beim Einführen /Durchführen des Gewindeteils durch das Durchgangsloch (an der Oberseite) teilweise oder vollständig passieren zu lassen.

Die Eindeutigkeit/Offensichtlichkeit bzw. die Ausschließlichkeit der Einführrichtung erleichtert in vorteilhafter Weise die Montage der beiden Kopfstücke zu einem Kopfteil und verhindert eine fehlerhafte Zusammensetzung der beiden Kopfstücke.

Außerdem bevorzugt hat das separate Kopfstück in Draufsicht eine im Wesentlichen Ellipsenform mit zwei sich gegenüberliegenden kleinradialen Endabschnitten und zwei sich gegenüberliegenden großradialen Längsabschnitten, die zumindest längsabschnittsweise als zueinander parallele Geraden ausgeformt sind. Alternativ kann das Kopfstück in Draufsicht auch eine ellipsenähnliche Form einnehmen mit zwei sich gegenüberliegenden radialen Endabschnitten und zwei sich gegenüberliegenden vollständig geraden, parallelen Längsabschnitten.

Durch die in Draufsicht großradialen Längsabschnitte, die zumindest längsabschnittsweise als zueinander parallele Geraden ausgeformt sind, bzw. die vollständig geraden, parallelen Längsabschnitte kann der Kopfteil der Knochenschraube bzw. das separate Kopfstück im Gegensatz zu einer in Draufsicht vollständig runden Ausführung mit seiner Umgebung (dem Langloch bzw. dem Implantat) einen Linienkontakt eingehen und eine stabilere Anlageposition einnehmen.

Die Draufsicht ist hierbei als eine in Axialrichtung des Durchgangslochs in dem separaten Kopfstück verlaufende Blickrichtung auf die Oberseite zu verstehen.

Darüber hinaus bevorzugt nimmt das separate Kopfstück im Wesentlichen die Form einer plankonvexen Linse ein und ist die Oberseite plan. Man kann die äußere Form dieser plankonvexen Linse auch als längshalbiertes Ellipsoid und/oder als ein längshalbiertes, gleichförmiges Ei beschreiben. Auch eine Walnusshälfte ähnelt dieser Form.

Insbesondere hat das separate, nicht-rotationssymmetrische, als im Wesentlichen plankonvexe Linse geformte Kopfstück an seiner konvexen Seite eine (Unterseite in Form einer) Abflachung, die im Wesentlichen planparallel zur planen Oberseite ausgerichtet ist. Dadurch wird in vorteilhafterweise eine Auflage- bzw. Anlagefläche geschaffen, die dem separaten Kopfstück bzw. dem Kopfteil an seiner Umgebung eine größere Abstütz- bzw. Anlagefläche bietet.

Insbesondere bevorzugt weist die Knochenschraube axial zwischen dem Außengewinde des Gewindeteils und dem verbundenen Kopfstück einen gewindelosen Schaftabschnitt auf, der so bemessen ist, dass das Außengewinde des Gewindeteils und das Innengewinde des separaten Kopfstücks außer Eingriff sind, wenn das verbundene Kopfstück an dem Hinterschnitt oder in der Trichter- oder Tulpenform axialfest anliegt.

Der gewindelose Schaftabschnitt hat bevorzugt einen Durchmesser von 3mm, insbesondere bevorzugt den gleichen Durchmesser wie das Kerngewinde des Gewindeteils und besonders bevorzugt einen Durchmesser, der größer als 3mm und kleiner als der distale Durchgangslochdurchmessers ist.

Dadurch wird erreicht, dass das separate Kopfstück in der axialen Anlageposition mit dem verbunden Kopfstück relativ zu diesem bewegbar ist bzw. bleibt und in der Montageposition eine gelenkartige Verbindung am Kopfteil der Knochenschraube gewährleistet.

Bevorzugt geht das separate Kopfstück einen Linien- und/oder Flächen-Abstützkontakt mit dem Implantat längs der zwei sich in Draufsicht gegenüberliegenden, großradialen bzw. parallelen Längsabschnitte ein. Der vergrößerte Abstütz- bzw. Auflagekontakt sichert die Knochenschraube gegen Durchschrauben. Weiter wird eine (elastische) Verformung des Schraubenkopfes beim Anziehen der Knochenschraube durch den größeren Anlagekontakt an den beiden in Draufsicht großradialen bzw. parallelen Längsabschnitten verhindert oder zumindest vermindert. Das Risiko auf ein Verklemmen des Schraubendrehers im ggf. deformierten Schraubenkopf wird somit reduziert.

Ebenso kann das separate Kopfstück einen Linien- und/oder Flächen-Abstützkontakt mit dem Implantat an einem der zwei sich gegenüberliegenden kleinradialen Endabschnitte eingehen und somit den Abstütz- bzw. Auflagekontakt der Knochenschraube am Implantat vergrößern.

Weiter bevorzugt zeigen die beiden sich gegenüberliegenden Außenflächen / Außenseiten des separaten Kopfstücks, welche die kleinradialen Endabschnitte der (planen) Oberseite (an der der Gewindeteil eingeführt wird) mit der Unterseite verbinden, einen (zum Mittelpunkt des separaten Kopfstücks) konvexen Verlauf. Man kann auch sagen, dass diese Außenflächen jeweils die Gestalt einer konvexen Kufe bzw. Wippe einnehmen. Entlang dieser wippenartigen Kufen kann der Kopfteil der Knochenschraube in Bezug auf das Implantat eine weitere Neigung eingehen. Der Ausrichtung der Knochenschraube kommt damit noch mehr Platzierungsspielraum zu.

Darüber hinaus kann die konvexe Fläche vorteilhaft als vergrößerte Anlagefläche zum in Kontakt treten des Kopfteils mit dem Implantat genutzt werden. Dabei ist es insbesondere vorteilhaft, wenn die Außenfläche des separaten Kopfstücks an dessen Querseiten (oder an mindestens einer Querseite), also den Flächen, die die kleinradialen Endabschnitte der (planen) Oberseite mit der Unterseite verbinden, komplementär zu mindestens einer Querseite des Langlochs bzw. der Innenumfangsfläche einer Querseite des Langlochs ausgebildet sind. Dies vergrößert vorteilhafterweise weiter den Flächenabstützkontakt und stabilisiert die Anlage des Kopfteils am Implantat.

Besonders bevorzugt ist am Innenumfang bzw. an der Innenumfangsseite des implantatseitigen Durchgangslochs bzw. Langlochs eine umlaufende Anlagefläche bzw. ein umlaufender Vorsprung ausgebildet, an welcher/welchem sich das separate Kopfstück derart abstützt, dass das separate Kopfstück und/oder das verbundene Kopfstück in seiner/ihrer Anlageposition am / im Implantat(loch) nicht über den inneren Umfangsrand des Durchgangslochs bzw. Langlochs (der dem Patientenknochen abgewandte und/oder einem Inlay zugewandte Umfangsrand) axial vorragt. In anderen Worten kann das Durchgangsloch bzw. Langloch einen hochgezogenen Rand aufweisen, der in Richtung seiner dem Patientenknochen zugewandten umlaufenden Kante eine (in das Durchgangsloch bzw. Langloch hineinragende) umlaufende, im Wesentlichen senkrecht zur Innenumfangsseite des Durchgangslochs bzw. Langlochs ausgerichtete oder zur Senkrechten der Innenumfangsseite des Durchgangslochs bzw. Langlochs geneigt ausgerichtete Anlagefläche für das separate Kopfstück ausbildet und wobei die Höhe des Durchganglochs (die Gesamthöhe der Anlagefläche und der Innenumfangsseite) vom Kopfteil der Knochenschraube in sämtlichen Auslenkpositionen des verbundenen Kopfstücks relativ zum separaten Kopfstück nicht überschritten wird.

Dadurch, dass das verbundene Kopfstück in keiner Auslenkposition bezüglich des separaten Kopfstücks aus dem Durchgangsloch bzw. Langloch heraus in das Innere (zu der dem Patientenknochen abgewandten Seite) des Implantats hineinragt wird erreicht, dass ein in dem Implantat vorgesehenes Inlay nicht auf dem Kopfteil der Knochenschraube aufsitzt.

Weiter bevorzugt kann das Durchgangsloch des separaten Kopfstücks derart ausgebildet sein, dass in montiertem Zustand des Kopfteils das verbundene Kopfstück, insbesondere in sämtlichen Auslenkpositionen relativ zum separaten Kopfstück, vollständig im Durchgangsloch des separaten Kopfstücks versenkt ist.

Dadurch kann in vorteilhafter Weise erreicht werden, dass die Höhe des Kopfteils (seine Länge in Axialrichtung des Durchgangslochs) an der Oberseite des separaten Kopfstücks nicht überschritten wird, insbesondere in allen Auslenkpositionen des verbundenen Kopfstücks relativ zum separaten Kopfstück nicht überschritten wird. Im Falle einer planen Oberseite des separaten Kopfstücks ist also das proximale Ende der Knochenschraube in montiertem Zustand des Kopfteils, insbesondere in sämtlichen Auslenkpositionen des verbundenen Kopfstücks relativ zum separaten Kopfstück, (stets) plan.

### Kurzbeschreibung der Zeichnungen

Die Erfindung wird nachstehend anhand bevorzugter Ausführungsbeispiele unter Bezugnahme auf die beigefügte Zeichnung näher beschrieben. Es zeigen:
Fig. 1 eine perspektivische Ansicht einer Knochenschraube in einer Ausführungsform der Erfindung
Fig. 2 eine perspektivische Ansicht eines separaten Kopfstücks in einer Ausführungsform der Erfindung
Fig. 3 eine perspektivische Ansicht einer montierten Knochenschraube in einer Ausführungsform der Erfindung
Fig. 4 einen Längsschnitt des Kopfteils der Knochenschraube entlang der Linie IV der Fig. 3
Fig. 5 einen Querschnitt des Kopfteils der Knochenschraube entlang der Linie V der Fig. 3
Fig. 6 eine perspektivische Innenansicht eines Implantats mit einer Knochenschraube in einer Ausführungsform der Erfindung
Fig. 7 eine perspektivische Außenansicht des Implantats der Fig. 6

Die Figuren sind lediglich schematischer Natur und dienen ausschließlich dem Verständnis der Erfindung. Die gleichen Elemente sind mit denselben Bezugszeichen bezeichnet.

### Detaillierte Beschreibung bevorzugter Ausführungsbeispiele

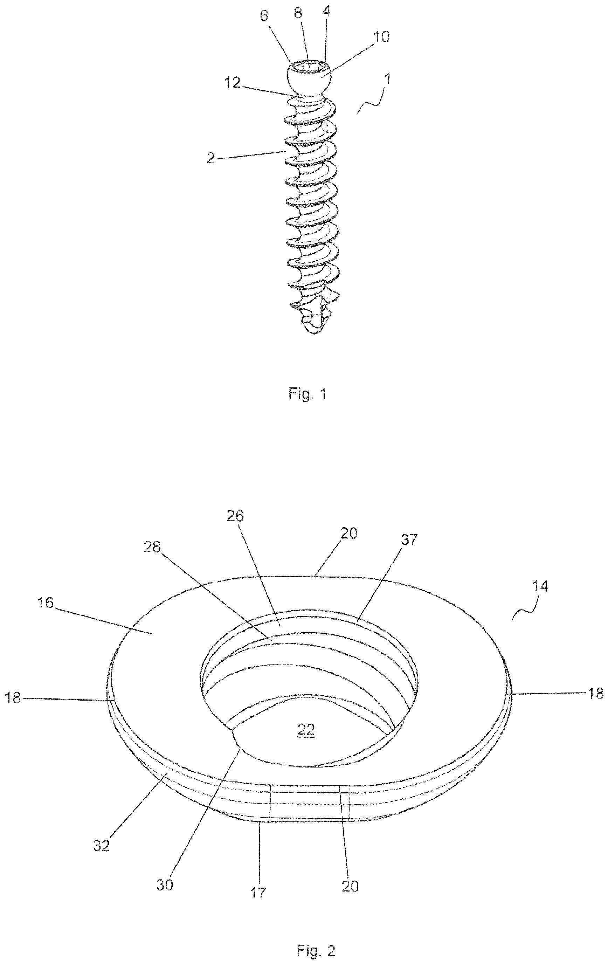

Fig. 1 zeigt eine medizinische Knochenschraube 1 mit einem schaftförmigen Gewindeteil 2 und einem rotationssymmetrischen, stoffeinstückig mit dem Gewindeteil 2 verbundenen Kopfstück 4. Das verbundene Kopfstück 4 weist eine plane (axiale) Oberseite 6 mit einer über diese in das Kopfstück 4 eingelassene Anschlussgeometrie 8 in Form eines Sechsecks zur Interaktion mit einem Montagewerkzeug auf. Es können alternativ sämtliche andere bekannte Anschlussgeometrien wie Schlitze, Kreuzschlitze oder Sterne realisiert sein. Weiter zeigt das verbundene Kopfstück 4 eine nach Außen konvex ausgebildete Außenumfangsseite 10, die am proximalen Ende des Kopfstücks 4 (an der umlaufenden Außenkante der Oberseite 6) einen größeren Durchmesser als am distalen Ende des Kopfstücks 4 hat. Das Kopfstück 4 nimmt eine teilkugelförmige Gestalt ein, deren größter Durchmesser zwischen dem proximalen Ende des Kopfstücks 4 und dem distalen Ende des Kopfstücks 4 liegt. Am distalen Ende des Kopfstücks 4 ist ein Übergangsabschnitt 12 angeordnet, der das Kopfstück 4 mit dem Gewindeteil 2 verbindet. Der Übergansabschnitt 12 ist gewindelos und weist einen Durchmesser auf, der die Größe des Kerndurchmessers des Gewindeteils 2 hat. Es ist auch denkbar, dass der Durchmesser des Übergangsabschnitts 12 größer als der Kerndurchmesser ist.

Fig. 2 stellt eine perspektivische Ansicht eines separaten Kopfstücks 14 dar. Das separate Kopfstück 14 hat die geometrische Gestalt einer plankonvexen Linse mit einer planen Oberseite 16 und weist weiter eine zu dieser im Wesentlichen parallele, plane Unterseite 17 auf. Die plane Oberseite 16 des separaten Kopfstücks 14 nimmt dabei in Draufsicht eine im Wesentlichen elliptische Form ein und weist zwei sich gegenüberliegende runde bzw. teilkreisbogenförmige Umfangskanten 18 und zwei sich gegenüberliegende, im Wesentlichen parallel verlaufende Umfangskanten 20 auf. Durch den plankonvexen Körper des separaten Kopfstücks 14 verläuft ein Durchgangsloch 22 von der Mitte der Oberseite 16 her und erstreckt sich im Wesentlichen senkrecht zur Oberseite 16 durch den plankonvexen Körper hindurch. Das Durchgangsloch 22 ist dabei rotationssymmetrisch geformt und zeigt an der gemeinsamen Kante mit der Oberseite 16 einen größeren Durchmesser als an der gemeinsamen Kante mit der im Wesentlichen planen Unterseite 17 des separaten Kopfstücks 14. Die Innenumfangsseite 26 des Durchgangslochs 22 nimmt also eine konkave und/oder im Wesentlichen, trichterförmige Gestalt ein. Die gemeinsame Kante des Durchgangslochs 22 mit der Oberseite 16 weist eine umlaufende Fase 37 auf. An der Innenumfangsseite 26 ist ein Innengewinde 28 in den plankonvexen Körper des Kopfstücks 14 eingeschnitten, das am Gewindeauslauf zur Oberseite 16 hin eine Ausnehmung 30 zeigt. Die Außenumfangsseite 32 des plankonvexen Körpers hat sowohl in Querrichtung als auch in Längsrichtung des Kopfstücks 14 an der Oberseite 16 einen breiteren Querschnitt als an der Unterseite 17 und zeigt insgesamt eine konvexe Form.

Unter Längsrichtung des Kopfstücks 14 ist die Richtung seiner längsten Ausdehnung in Draufsicht und unter Querrichtung die Richtung seiner kürzesten Ausdehnung in Draufsicht zu verstehen

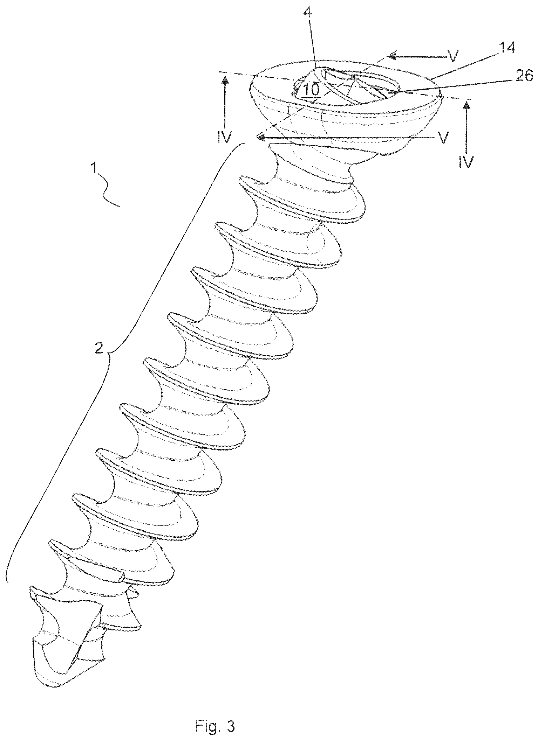

Fig. 3 zeigt eine perspektivische Ansicht einer Knochenschraube 1, in der das mit dem Gewindeteil 2 verbundene Kopfstück 4 und das separate Kopfstück 14 zu einem Kopfteil 34 montiert sind. In der dargestellten Position ist die Außenumfangsseite 10 des verbundenen Kopfstücks 4 mit der Innenumfangsseite 26 des separaten Kopfstücks 14 bewegbar in Anlage gebracht. Das Kopfteil nimmt in der Montageposition die Funktion eines Kugelgelenks ein, bei der das separate Kopfstück 14 nach Art einer Kugelpfanne und das Kopfstück 4 nach Art eines Gelenks gebildet sind und das separate Kopfstück 14 das verbundene Kopfstück 4 derart umschließt, dass eine rotatorische Bewegung der beiden Kopfstücke 4, 14 gegeneinander möglich ist, eine Axialbewegung der beiden Kopfstücke relativ zueinander aber stark begrenzt ist oder nicht möglich ist. Die Dimension der Innengeometrie des Kopfstücks 14 ist komplementär zur Außengeometrie des Kopfstücks 4. Die Montage der beiden Kopfstücke 4, 14 erfolgt durch Eindrehen des Gewindeteils 2 in das bzw. durch das separate Kopfstück 14 hindurch, bis der Gewindeteil 2 das separate Kopfstück 14 vollständig passiert hat. Das Innengewinde 28 (nicht sichtbar) des separaten Kopfstücks 14 entspricht dem Außengewinde des Gewindeteils 2.

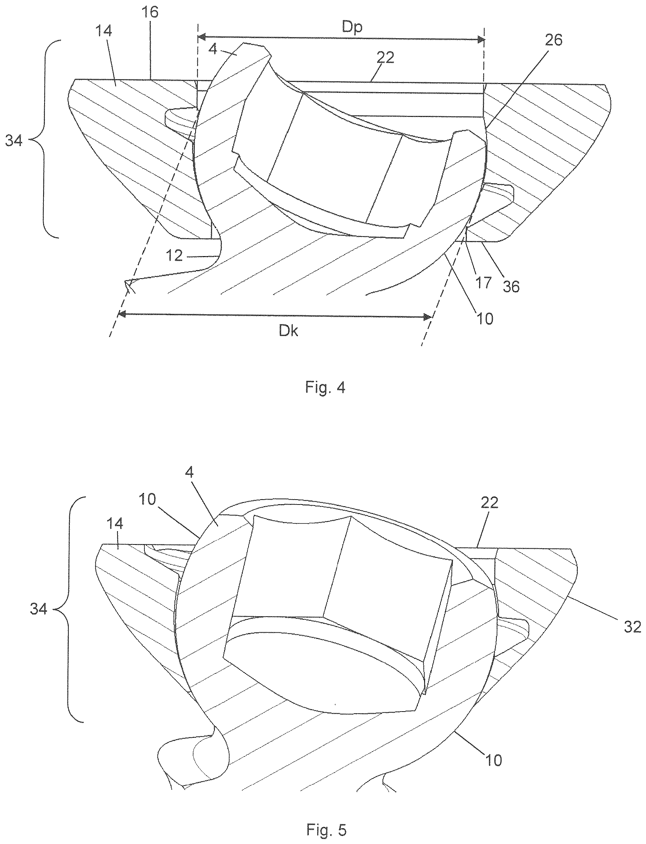

Fig. 4 zeigt einen Längsschnitt des Kopfteils 34 bestehend aus dem separaten Kopfstück 14 und dem verbundenen Kopfstück 4 entlang der Linie IV der Fig. 3. Das verbundene Kopfstück 4 ist gegenüber dem separaten Kopfstück 14 maximal ausgelenkt, d.h. das Kopfstück 4 bzw. der Gewindeteil 2 befindet sich in Bezug zur Unterseite 17 in seiner maximal möglichen Neigungsposition (siehe linke Seite in der Figur). Die gemeinsame Kante der Unterseite 17 und des Durchgangslochs 22 befindet sich in dieser Neigungsposition auf der einen Seite des separaten Kopfstücks 14 im Übergangsabschnitt 12 und auf der andere Seite an der Außenumfangsseite 10 des verbundenen Kopfstücks 4. Die komplementäre Gestalt der Außenumfangsseite 10 und der Innenumfangsseite 26 erlaubt den Kopfstücken 4, 14 aneinander entlang zu gleiten und gegeneinander auslenkbar zu sein, während die Kopfstücke 4, 14 axialfest aneinander anliegen. Die Unterseite 17 weist zwischen dem Durchgangsloch 22 und ihrer umlaufen Außenkante einen planen Flächenabschnitt 36 auf. Das Durchgangsloch 22 weist an seinem proximalen Ende einen proximalen Durchgangslochdurchmesser Dp auf. Das verbundene Kopfstück 4 weist einen Kopfstückaußendurchmesser Dk auf. Es ist zu sehen, dass der proximale Durchgangslochdurchmesser Dp schmaler als der Kopfstückaußendurchmesser Dk ist. Dabei weist der proximale Durchgangslochdurchmesser Dp eine Fase 37 auf, deren Durchmesser zur Oberseite 16 hin zunimmt und dabei den proximalen Durchgangslochdurchmesser Dp überschreitet.

Fig. 5 zeigt einen Querschnitt des Kopfteils 34 bestehend aus dem separaten Kopfstück 14 und dem verbundenen Kopfstück 4 entlang der Linie V der Fig. 3. Das verbundene Kopfstück 4 ist gegenüber dem separaten Kopfstück 14 maximal ausgelenkt, d.h. das Kopfstück 4 bzw. der Gewindeteil 2 befindet sich in Bezug zur Außenumfangsseite 32 in seiner maximal möglichen Neigungsposition (siehe linke Seite in der Figur). In der Querrichtung des separaten Kopfstücks 14 bilden das Durchgangsloch 22 und die Außenumfangsseite 32 des separaten Kopfstücks 14 eine gemeinsame Kante, an der die Außenumfangsseite 10 des verbundenen Kopfstücks 4 anliegt und sich bei einer Auslenkungsbewegung der Kopfstücke 4, 14 an dieser entlang bewegt.

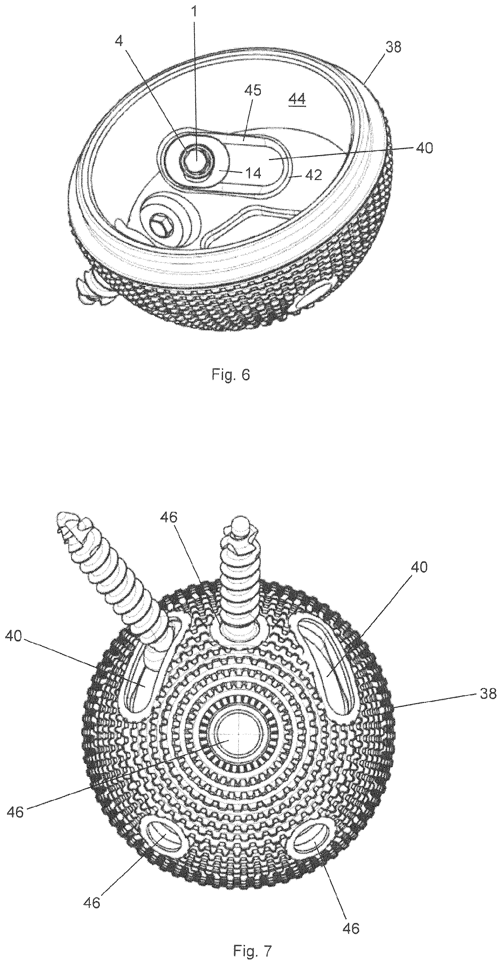

Fig. 6 zeigt eine perspektivische Innenansicht eines Implantats 38 mit einer Knochenschraube 1 in einer Ausführungsform der Erfindung. Das Implantat 38 ist ein Pfannenimplantat, beispielsweise für den Einsatz als Hüftgelenkspfannenimplantat. In das Implantat 38 ist zumindest ein Langloch 40 eingebracht, dessen Breite (quer zu seiner Längsachse) auf den Durchmesser des Gewindeteils 2 und auf die Quermaße des separaten Kopfstücks 14 abgestimmt sind. Das Langloch 40 hat entlang seiner Längsachse zwei gegenüberliegende parallele Längsseiten, an seinen Enden quer zur Längsachse zwei gegenüberliegende runde Querseiten und bildet entlang seines Innenumfangs eine Innenumfangsseite 42 aus. Das Implantat 38 hat eine Innenseite 44, über welche eine Knochenschraube 1 in das Langloch 40 eingeführt werden kann.

Die Innenumfangsseite 42 hat an ihrem proximalen Ende eine umlaufende gemeinsame Kante mit der Innenseite 44 und weist an ihrem distalen Ende eine umlaufende Anlagefläche 45 auf, an der das separate Kopfstück 14 anliegt bzw. an der es sich abstützt. Die Anlagefläche 45 ist bezüglich der Innenumfangsseite 42 geneigt ausgebildet und erstreckt sich von der gemeinsamen Kante mit der Innenumfangsseite 42 zur Außenseite des Implantats 38 hin und verjüngt dabei das Durchgangslochs 22. Man kann auch sagen, dass der Durchmesser des Durchgangslochs 22 sowohl in Quer- als auch in Längsrichtung des Langlochs 40 entlang der Anlagefläche 45 zur Außenseite des Implantats 38 hin (linear und/oder exponentiell) abnimmt.

Das separate Kopfstück 14 liegt in dem gezeigten montierten Zustand an der Anlagefläche 45 und der Innenumfangsseite 42 an und steht dadurch an seinen Längsseiten und an einer seiner Querseiten in Flächenanlagekontakt mit dem Implantat 38. Die Gesamthöhe der Innenumfangsseite 42 und der Anlagefläche 45, also der Abstand der Innenseite 44 und der Außenseite des Implantats 38 in Durchtrittsrichtung der Knochenschraube 1 durch das Langloch 40, ist dabei so gewählt, dass in montiertem Zustand der Knochenschraube 1 mit dem Implantat 38 der Kopfteil 34 (in sämtlichen Positionen der beiden Kopfstücke 4, 14 zueinander) nicht über die Innenseite 44 hinausragt bzw. der Kopfteil 34 in montiertem Zustand der Knochenschraube 1 mit dem Implantat 38 vollständig im Langloch 40 versenkt ist. Entlang der Längsachse des Langlochs 40 kann die Knochenschraube 1 je nach gewünschter Eintrittsposition in den Patientenknochen angeordnet werden.

Die Innenumfangsseite 42 und/oder die Anlagefläche 45 des Langlochs 40 ist / sind dabei an dessen / deren Querseiten komplementär zu den Außenflächen des separaten Kopfstücks 14 an dessen Querseiten gebildet. Ebenso ist / sind die Innenumfangsseite 42 und/oder die Anlagefläche 45 des Langlochs 40 an dessen / deren Längsseiten komplementär zu den Außenflächen des separaten Kopfstück 14 an dessen Längsseiten gebildet. Insgesamt nimmt das Langloch 40 bzw. nehmen die Innenumfangsseite 42 und die Anlagefläche 45 insgesamt also eine (im Wesentlichen) wannenförmige oder wannenartige Gestalt ein, die (zumindest teilweise) komplementär zur Außenumfangsseite 32 des separaten Kopfstücks 14 ist, sodass das separate Kopfstück 14 an der Anlagefläche 45 und/oder der Innenumfangsseite 42 des Langlochs 40 einen Flächenanlagekontakt ausbildet.

Fig. 7 zeigt eine perspektivische Außenansicht des Implantats der Fig. 6. Es ist zu sehen, dass das Implantat 38 zwei Langlöcher 40 und mehrere runde Löcher 46 aufweist.

Es wird angemerkt, dass anstelle eines Langlochs auch ein rundes Durchgangsloch mit gleichen Eigenschaften vorgesehen sein kann. Anstelle der Längs- und Querseiten liegen bei einem runden Durchgangsloch gleichradiale Seiten vor. Dabei kann das separate Kopfstück eine im Wesentlichen halbkugelartige Gestalt mit einer planen, in Draufsicht runden Oberseite und einer dazu im Wesentlichen parallelen Unterseite aufweisen. Das runde Durchgangsloch kann eine umlaufende Anlagefläche und/oder eine Innenumfangsfläche aufweisen, die komplementär zur halbkugelartigen Gestalt des separaten Kopfstücks geformt ist/sind, sodass das separate Kopfstück und die umlaufende Anlagefläche des runden Durchgangslochs in montiertem Zustand aneinander anliegen.

In anderen Worten zusammengefasst handelt es sich um ein Implantatsystem mit einem Hüftgelenks-Pfannenimplantat und einer Knochenschraube mit gelenkigem Schraubenkopf, der bei einer Ausrichtung der Knochenschraube relativ zum Pfannenimplantat eine unveränderte Anlageposition des Schraubenkopfs am Pfannenimplantat ermöglicht. Hierfür ist im Pfannenimplantat ein Langloch ausgebildet, in welchem die Knochenschraube in Längsrichtung des Langlochs verschwenkbar ist.

### Bezugszeichenliste

- 1: Knochenschraube
- 2: Gewindeteil
- 4: verbundenes Kopfstück
- 6: Oberseite des Kopfstücks 4
- 8: Anschlussgeometrie
- 10: Außenumfangsseite des Kopfstücks 4
- 12: Übergangsabschnitt
- 14: separates Kopfstück
- 16: Oberseite des Kopfstücks 14
- 17: Unterseite des Kopfstücks 14
- 18: abgerundete Umfangskanten der Oberseite 16
- 20: parallele Umfangskanten der Oberseite 16
- 22: Durchgangsloch
- 26: Innenumfangsseite des Durchgangslochs 22
- 28: Innengewinde des separaten Kopfstücks 14
- 30: Ausnehmung
- 32: Außenumfangsseite des separaten Kopfstücks 14
- 34: Kopfteil der Knochenschraube 1
- 36: planer Flächenabschnitt der Unterseite 17
- 37: Fase
- 38: Implantat
- 40: Durchgangsloch/Langloch
- 42: Innenumfangsseite des Durchgangslochs/Langlochs 40
- 44: Innenseite des Implantats 38
- 45: Anlagefläche
- 46: rundes Loch
- Dp: proximaler Durchgangslochdurchmesser
- Dk: Kopfstückaußendurchmesser

## Patentansprüche

1. Implantatsystem mit mindestens einer medizinischen Knochenschraube (1) und einem Hüftgelenkspfannenimplantat (38), das mindestens ein Durchgangsloch (40) aufweist, durch welches die medizinische Knochenschraube (1) derart einführbar ist, dass die medizinische Knochenschraube (1) zumindest in eine Richtung des Durchgangslochs (40) schwenkbar ist, wobei
die medizinische Knochenschraube (1) zur Befestigung des Hüftgelenkspfannenimplantats (38) an einem Patientenknochen einen schaftförmigen Knochengewindeteil (2) aufweist, der einen Kerndurchmesser und einen Außengewindedurchmesser hat und an dessen proximalem Ende sich ein zweigeteilter, winkelbarer Kopfteil (34) anschließt,
der zweigeteilte, winkelbare Kopfteil (34) aus einem einstückig mit dem Knochengewindeteil (2) ausgebildeten, vorzugsweise rotationssymmetrischen, weiter vorzugsweise teilkugelförmigen Kopfstück (4) und einem separat zu dem Knochengewindeteil (2) vorgesehenen Kopfstück (14) gebildet ist,
das separat zu dem Knochengewindeteil (2) vorgesehene Kopfstück (14) eine Oberseite (16) und eine Unterseite (17) aufweist und ein Durchgangsloch (22) mit einem Hinterschnitt oder einer Trichter- oder Tulpenform in dieses eingebracht ist, und
das Durchgangsloch (22) einen proximalen Durchgangslochdurchmesser (Dp) an der Oberseite (16) und einen distalen Durchgangslochdurchmesser an der Unterseite (17) hat,
**dadurch gekennzeichnet, dass**
das Durchgangsloch (22) weiter zwischen der Oberseite (16) und der Unterseite (17) ein dem Außengewinde des schaftförmigen Knochengewindeteils (2) entsprechendes Innengewinde (28) mit einem Innengewindedurchmesser hat,
der Innengewindedurchmesser größer als der Außengewindedurchmesser und der distale Durchgangslochdurchmesser größer als der Kerndurchmesser ist, und
der schaftförmige Knochengewindeteil (2) durch Einschrauben des schaftförmigen Knochengewindeteils (2) in das Durchgangsloch (22) derart in das separat zu dem Knochengewindeteil vorgesehene Kopfstück (14) eingeführt ist, dass das einstückig mit diesem ausgebildete Kopfstück (4) an dem Hinterschnitt oder in der Trichter- oder Tulpenform axialfest anliegt, jedoch eine Schwenkbewegung des schaftförmigen Knochengewindeteils (2) bezüglich des separaten Kopfstücks (14) in zumindest einer oder ausschließlich einer Schwenkebene zugelassen ist.

2. Implantatsystem nach Anspruch 1, **dadurch gekennzeichnet, dass** das Durchgangsloch (40) ein Langloch ist, welches zwei sich gegenüberliegende lange Längsseiten und zwei sich gegenüberliegende kurze Querseiten hat, durch welches die medizinische Knochenschraube (1) derart einführbar ist, dass die medizinische Knochenschraube (1) zumindest in Längsrichtung des Langlochs schwenkbar ist.

3. Implantatsystem nach Anspruch 1 oder 2, **dadurch gekennzeichnet, dass** das einstückig mit dem Knochengewindeteil (2) ausgebildete Kopfstück (4) einen Kopfstückaußendurchmesser (Dk) hat und das Durchgangsloch (22) einen intermediären Durchgangslochdurchmesser zwischen der Oberseite (16) und der Unterseite (17) hat, der größer, gleich oder kleiner als der Kopfstückaußendurchmesser (Dk) ist.

4. Implantatsystem nach einem der vorstehenden Ansprüche, **dadurch gekennzeichnet, dass** der proximale Durchgangslochdurchmesser (Dp) kleiner als der Kopfstückaußendurchmesser (Dk) ist.

5. Implantatsystem nach einem der vorstehenden Ansprüche, **dadurch gekennzeichnet, dass** der proximale Durchgangslochdurchmesser (Dp) größer als der distale Durchgangslochdurchmesser ist und/oder der Hinterschnitt oder die Trichter- oder Tulpenform so ausgebildet und/oder ausgerichtet ist, dass sich zwangsläufig eine Einführrichtung des schaftförmigen Knochengewindeteils (2) von der Oberseite (16) aus ergibt.

6. Implantatsystem nach einem der vorstehenden Ansprüche, **dadurch gekennzeichnet, dass** das separate Kopfstück (14) in Draufsicht im Wesentlichen eine Ellipsenform hat mit zwei sich gegenüberliegenden kleinradialen Endabschnitten und zwei sich gegenüberliegenden großradialen Längsabschnitten, die zumindest längsabschnittsweise als zueinander parallele Geraden ausgeformt sind.

7. Implantatsystem nach einem der vorstehenden Ansprüche, **dadurch gekennzeichnet, dass** das separate Kopfstück (14) im Wesentlichen die Form einer plankonvexen Linse einnimmt und die Oberseite (16) plan ist.

8. Implantatsystem nach Anspruch 7, **dadurch gekennzeichnet, dass** das separate, als im Wesentlichen plankonvexe Linse geformte Kopfstück (14) an seiner konvexen Seite eine Abflachung hat, die im Wesentlichen planparallel zur planen Oberseite (16) ausgerichtet ist.

9. Implantatsystem nach einem der vorstehenden Ansprüche, **dadurch gekennzeichnet, dass** die Knochenschraube (1) axial zwischen dem Außengewinde des Knochengewindeteils (2) und dem einstückig mit diesem ausgebildeten Kopfstück (4) einen gewindelosen Schaftabschnitt (12) aufweist, der so bemessen ist, dass das Außengewinde des Knochengewindeteils (2) und das Innengewinde (28) des separaten Kopfstücks (14) außer Eingriff sind, wenn das einstückig mit dem Knochengewinde (2) ausgebildete Kopfstück (4) an dem Hinterschnitt oder in der Trichter- oder Tulpenform axialfest anliegt.

10. Implantatsystem nach einem der Ansprüche 6 bis 9, **dadurch gekennzeichnet, dass** das separate Kopfstück (14) einen Linien- oder Flächen-Abstützkontakt mit dem Implantat (38) längs der zwei sich gegenüberliegenden großradialen Längsabschnitte eingeht.

11. Implantatsystem nach einem der Ansprüche 6 bis 10, **dadurch gekennzeichnet, dass** das separate Kopfstück (14) einen Linien- oder Flächen-Abstützkontakt mit dem Implantat (38) an einem der zwei sich gegenüberliegenden kleinradialen Endabschnitte eingehen kann.

12. Implantatsystem nach einem der Ansprüche 6 bis 11, **dadurch gekennzeichnet, dass** mindestens ein kleinradialer Endabschnitt des separaten Kopfstücks (14), bevorzugt beide kleinradiale Endabschnitte, komplementär zu mindestens einer Querseite des Durchgangslochs (40) ausgebildet ist/sind.

13. Implantatsystem nach einem der vorstehenden Ansprüche, **dadurch gekennzeichnet, dass** am Innenumfang des implantatseitigen Durchgangslochs (40) eine umlaufende Anlagekante (45) ausgebildet ist, an welcher sich das separate Kopfstück (14) derart abstützt, dass das stoffeinstückig mit dem Knochengewinde (2) ausgebildete Kopfstück (4) nicht über den Umfangsrand des Durchgangslochs (40) axial vorragt.

## Claims

1. Implant system comprising at least a medical bone screw (1) and a hip-joint acetabular implant (38) having at least one clearance hole (40) through which the medical bone screw (1) can be inserted in such a way that the medical bone screw (1) can be pivoted at least in one direction of the clearance hole (40), wherein
the medical bone screw (1) has a shaft-shaped bone thread part (2) for fastening the hip-joint acetabular implant (38) to a patient bone, which has a core diameter and an outer-thread diameter and at the proximal end of which a two-part, angle-adjustable head part (34) is connected,
the two-part, angle-adjustable head part (34) is formed from a head piece (4), which is formed in one piece with the bone-thread part (2), which is preferably rotationally symmetrical, more preferably partially spherical, and a head piece (14) provided separately from the bone-thread part (2),
the head piece (14) provided separately from the bone-thread part (2) has an upper side (16) and a lower side (17), and a clearance hole (22) having an undercut or a funnel shape or tulip shape is formed therein, and
the clearance hole (22) has a proximal clearance-hole diameter (Dp) at the upper side (16) and a distal clearance-hole diameter at the lower side (17),
**characterized in that**
the clearance hole (22) furthermore has an inner thread (28) with an inner-thread diameter between the upper side (16) and the lower side (17), the inner thread (28) corresponding to the outer thread of the shaft-shaped bone-thread part (2),
wherein the inner-thread diameter is greater than the outer-thread diameter and the distal clearance-hole diameter is greater than the core diameter, and
by screwing the shaft-shaped bone thread part (2) into the clearance hole (22), the shaft-shaped bone-thread part (2) is inserted into the head piece (14) provided separately from the bone-thread part, in such a way that the head piece (4) formed in one piece with the latter rests in an axially fixed manner on the undercut or in the funnel shape or tulip shape, but a pivoting movement of the shaft-shaped bone thread part (2) with respect to the separate head piece (14) is allowed in at least one or exclusively one pivoting plane.

2. Implant system according to claim 1, **characterized in that** the clearance hole (40) is a slotted hole having two opposite, long longitudinal sides and two opposite, short transverse sides, wherein the medical bone screw (1) is insertable in such a way through the slotted hole that the medical bone screw (1) is pivotable at least in the longitudinal direction of the slotted hole.

3. Implant system according to claim 1 or 2, **characterized in that** the head piece (4) formed in one piece with the bone-thread part (2) has a head piece outer diameter (Dk) and the clearance hole (22) has an intermediary clearance-hole diameter between the upper side (16) and the lower side (17) which is greater than, equal to, or smaller than the head piece outer diameter (Dk).

4. Implant system according to one of the preceding claims, **characterized in that** the proximal clearance-hole diameter (Dp) is smaller than the head piece outer diameter (Dk).

5. Implant system according to one of the preceding claims, **characterized in that** the proximal clearance-hole diameter (Dp) is larger than the distal clearance-hole diameter and/or the undercut or the funnel shape or tulip shape is designed and/or oriented such that an insertion direction of the shaft-shaped bone thread part (2) from the upper side (16) inevitably results.

6. Implant system according to one of the preceding claims, **characterized in that** the separate head piece (14) in top view has a substantially elliptical shape with two opposite end portions with small radii and two opposite longitudinal portions with large radii, which are formed at least in longitudinal sections as parallel straight lines.

7. Implant system according to one of the preceding claims, **characterized in that** the separate head piece (14) substantially assumes the shape of a plano-convex lens and that the upper side (16) is planar.

8. Implant system according to claim 7, **characterized in that** the separate head piece (14) formed as a substantially plano-convex lens has a flattening on its convex side which is oriented substantially plane-parallel to the planar upper side (16).

9. Implant system according to one of the preceding claims, **characterized in that** the bone screw (1) has a threadless shaft portion (12) arranged axially between the outer thread of the bone-thread part (2) and the head piece (4) formed in one piece therewith, said threadless shaft portion (12) being dimensioned in such a way that the outer thread of the bone-thread part (2) and the inner thread (28) of the separate head piece (14) are disengaged when the head piece (4), which is formed in one piece with the bone thread (2), lies in an axially fixed manner against the undercut or in the funnel shape or tulip shape.

10. Implant system according to one of claims 6 to 9, **characterized in that** the separate head piece (14) establishes a line or surface supporting contact with the implant (38) along the two opposite longitudinal portions with large radii.

11. Implant system according to one of claims 6 to 10, **characterized in that** the separate head piece (14) can establish a line or surface supporting contact with the implant (38) at one of the two opposite end portions with small radii.

12. Implant system according to one of claims 6 to 11, **characterized in that** at least one end portion with small radius of the separate head piece (14), preferably both end portions with small radii, is/are formed complementary to at least one transverse side of the clearance hole (40).

13. Implant system according to one of the preceding claims, **characterized in that** a circumferential contact edge (45) is formed on the inner circumference of the implant side clearance hole (40), on which the separate head piece (14) is supported in such a way that the head piece (4) formed in one piece with the bone thread (2) does not project axially beyond the circumferential edge of the clearance hole (40).

## Revendications

1. Système d'implant avec au moins une vis d'ostéosynthèse médicale (1) et un implant acétabulaire d'articulation de la hanche (38), qui présente au moins un orifice de passage (40), à travers lequel la vis d'ostéosynthèse médicale (1) peut être introduite de telle manière que la vis d'ostéosynthèse médicale (1) peut pivoter au moins dans une direction de l'orifice de passage (40), dans lequel
la vis d'ostéosynthèse médicale (1) présente une partie filetée osseuse (2) en forme de tige pour la fixation de l'implant acétabulaire d'articulation de la hanche (38) au niveau d'un os du patient, qui a un diamètre intérieur et un diamètre de filetage extérieur et qui se raccorde au niveau de son extrémité proximale à une partie de tête divisée en deux pouvant former un angle (34),
la partie de tête divisée en deux pouvant former un angle (34) est constituée d'un élément de tête (4) réalisé d'un seul tenant avec la partie filetée osseuse (2), de préférence à symétrie de révolution, plus préférentiellement partiellement sphérique et d'un élément de tête (14) prévu séparément par rapport à la partie filetée osseuse (2),
l'élément de tête (14) prévu séparément par rapport à la partie filetée osseuse (2) présente une face supérieure (16) et une face inférieure (17) et un orifice de passage (22) avec une contre-dépouille ou une forme d'entonnoir ou de tulipe qui est introduite dans celui-ci, et
l'orifice de passage (22) a un diamètre proximal de l'orifice de passage (Dp) au niveau de la face supérieure (16) et un diamètre distal de l'orifice de passage au niveau de la face inférieure (17),
**caractérisé en ce que**
l'orifice de passage (22) a en outre entre la face supérieure (16) et la face inférieure (17) un filetage intérieur (28) correspondant au filetage extérieur de la partie filetée osseuse (2) en forme de tige présentant un diamètre de filetage intérieur,
le diamètre du filetage intérieur est supérieur au diamètre du filetage extérieur et le diamètre distal de l'orifice de passage est supérieur au diamètre intérieur, et
la partie filetée osseuse (2) en forme de tige par vissage de la partie filetée osseuse (2) en forme de tige dans l'orifice de passage (22) est introduite dans l'élément de tête (14) prévu séparément par rapport à la partie filetée osseuse de telle manière que l'élément de tête (4) réalisé d'un seul tenant avec celle-ci s'appuie de manière fixe axialement sur la contre-dépouille ou dans la forme d'entonnoir ou de tulipe, toutefois un mouvement pivotant de la partie filetée osseuse (2) en forme de tige par rapport à l'élément de tête (14) distinct est permis dans au moins un ou seulement un plan de pivotement.

2. Système d'implant selon la revendication 1, **caractérisé en ce que** l'orifice de passage (40) est un orifice oblong, lequel possède deux longues faces longitudinales opposées l'une à l'autre et deux faces transversales courtes opposées l'une à l'autre, par lequel la vis d'ostéosynthèse médicale (1) peut être introduite de telle manière que la vis d'ostéosynthèse médicale (1) peut pivoter au moins dans une direction longitudinale de l'orifice oblong.

3. Système d'implant selon la revendication 1 ou 2, **caractérisé en ce que** l'élément de tête (4) réalisé d'un seul tenant avec la partie filetée osseuse (2) a un diamètre extérieur de l'élément de tête (Dk) et l'orifice de passage (22) a un diamètre de l'orifice de passage intermédiaire entre la face supérieure (16) et la face inférieure (17), qui est supérieur, égal ou inférieur au diamètre extérieur de l'élément de tête (Dk).

4. Système d'implant selon l'une quelconque des revendications précédentes, **caractérisé en ce que** le diamètre proximal de l'orifice de passage (Dp) est inférieur au diamètre extérieur de l'élément de tête (Dk).

5. Système d'implant selon l'une quelconque des revendications précédentes, **caractérisé en ce que** le diamètre proximal de l'orifice de passage (Dp) est supérieur au diamètre distal de l'orifice de passage et/ou la contre-dépouille ou la forme d'entonnoir ou de tulipe est réalisée et/ou orientée de telle manière qu'il existe nécessairement un sens d'introduction de la partie filetée osseuse (2) en forme de tige à partir de la face supérieure (16).

6. Système d'implant selon l'une quelconque des revendications précédentes, **caractérisé en ce que** l'élément de tête (14) distinct a sensiblement en vue de dessus une forme elliptique avec deux sections d'extrémité de petit rayon opposées l'une à l'autre et deux sections longitudinales de grand rayon opposées l'une à l'autre, qui sont formées au moins sur certaines sections longitudinales en tant que droites parallèles les unes aux autres.

7. Système d'implant selon l'une quelconque des revendications précédentes, **caractérisé en ce que** l'élément de tête (14) distinct prend sensiblement la forme d'une lentille plan-convexe et la face supérieure (16) est plane.

8. Système d'implant selon la revendication 7, **caractérisé en ce que** l'élément de tête (14) distinct formé sensiblement comme lentille plan-convexe a au niveau de sa face convexe un aplatissement, qui est orienté sensiblement parallèlement au plan par rapport à la face supérieure (16) plane.

9. Système d'implant selon l'une quelconque des revendications précédentes, **caractérisé en ce que** la vis d'ostéosynthèse (1) présente une section de tige (12) non filetée axialement entre le filetage extérieur de la partie filetée osseuse (2) et l'élément de tête (4) réalisé d'un seul tenant avec celle-ci, dimensionnée de telle manière que le filetage extérieur de la partie filetée osseuse (2) et le filetage intérieur (28) de l'élément de tête (14) distinct ne sont pas en prise, lorsque l'élément de tête (14) réalisé d'un seul tenant avec le filetage osseux (2) s'appuie de manière fixe axialement sur la contre-dépouille ou dans la forme d'entonnoir ou de tulipe.

10. Système d'implant selon l'une quelconque des revendications 6 à 9, **caractérisé en ce que** l'élément de tête (14) distinct entre en contact d'appui linéaire ou superficiel avec l'implant (38) le long des deux sections longitudinales de grand rayon opposées l'une à l'autre.

11. Système d'implant selon l'une quelconque des revendications 6 à 10, **caractérisé en ce que** l'élément de tête (14) distinct peut entrer en contact d'appui linéaire ou superficiel avec l'implant (38) sur l'une des deux sections d'extrémité de petit rayon opposées l'une à l'autre.

12. Système d'implant selon l'une quelconque des revendications 6 à 11, **caractérisé en ce qu'**au moins une section d'extrémité de petit rayon de l'élément de tête (14) distinct, de préférence les deux sections d'extrémité de petit rayon, est/sont réalisée(s) de manière complémentaire par rapport à au moins une face transversale de l'orifice de passage (40).

13. Système d'implant selon l'une quelconque des revendications précédentes, **caractérisé en ce qu'**un bord d'appui périphérique (45) est réalisé au niveau de la périphérie intérieure de l'orifice de passage (40) côté implant, sur lequel l'élément de tête (14) distinct prend appui de telle manière que l'élément de tête (4) réalisé d'un seul tenant de matière avec le filetage osseux (2) ne fait pas saillie axialement sur le bord périphérique de l'orifice de passage (40).
